# FASCICULE DE BREVET EUROPEEN

(11) **EP 2 185 134 B1**
(45) Date de publication et mention de la délivrance du brevet: **22.11.2017**
(21) Numéro de dépôt: 08836034.2
(22) Date de dépôt: 05.09.2008
(51) Int. Cl.: A61K 9/16, A61K 31/167, A61K 31/192

(54) **DISPOSITIF POUR L'ADMINISTRATION PAR VOIE ORALE DE PRINCIPES ACTIFS**
VORRICHTUNG ZUR ORALEN VERABREICHUNG VON WIRKSTOFFEN
DEVICE FOR THE ORAL ADMINISTRATION OF ACTIVE PRINCIPLES

(30) Priorité: 05.09.2007 FR 0757364
(43) Date de publication de la demande: 19.05.2010
(73) Titulaire: Unither Pharmaceuticals, 80000 Amiens (FR)
(72) Inventeur: MAURY, Marc, F-33160 Saint Medard en Jalles (FR)
(74) Mandataire: Aquinov
(86) Numéro de dépôt international: PCT/FR2008/051590
(87) Numéro de publication internationale: WO 2009/044056

(56) Documents cités:
- EP-A- 0 383 503
- WO-A-94/09762
- WO-A-03/101226
- WO-A-2006/097361
- WO-A-2008/036885
- DE-A1- 3 731 058
- US-A- 5 270 056
- US-A- 5 718 681
- US-A1- 2005 220 874
- US-A1- 2006 147 497
- US-A1- 2008 181 932

## Description

La présente invention concerne un dispositif pour l'administration de médicaments par voie orale, permettant de témoigner de la prise des médicaments dispensés.

La prise de médicaments par les enfants et les personnes âgées pose de nombreux problèmes.

En effet, il est extrêmement difficile de leur administrer par ingestion orale des formes pharmaceutiques solides classiques comme des comprimés ou des gélules. Par ailleurs, si les formes liquides telles que des sirops ou des solutés buvables, sont plus faciles d'utilisation, elles présentent également des inconvénients techniques importants.

Tout d'abord de nombreux principes actifs ne peuvent pas être présentés sous forme liquide, pour des problèmes de solubilité, de stabilité ou de goût. La stabilité des médicaments présentés sous une forme liquide est souvent difficile à maîtriser car les processus de dégradation, d'hydrolyse sont fortement accrus en présence d'eau.

De plus, la formulation d'un produit pharmaceutique liquide nécessite généralement l'utilisation d'agents conservateurs anti-microbiens difficiles à maîtriser et conférant des goûts désagréables et rémanents.

En outre, pour s'assurer que le patient prenne bien la dose du médicament, il est nécessaire de réduire au maximum le volume de soluté délivré. Classiquement, les volumes de soluté pour un adulte sont voisins de 15 ml, ce qui correspond à une cuillère à soupe, et pour un enfant de 5ml, soit l'équivalent d'une cuillère à café. Or, avec un volume aussi faible il est difficile de maîtriser la dissolution des principes actifs ainsi que le goût de la préparation. On peut notamment citer le cas des préparations à base d'ibuprofène ou de paracétamol qui sont souvent présentées sous forme de suspension aqueuse dont la stabilité physico-chimique est prise en défaut à cause de problèmes de recristallisation.

Pour toutes ces raisons, malgré leur intérêt évident pour les patients, les formes pharmaceutiques liquides à usage oral ne sont pas satisfaisantes.

Afin de pallier aux problèmes de formulations de certaines formes pharmaceutiques à usage oral, il existe des formes pharmaceutiques solides devant être solubilisées avant ingestion par les patients. C'est le cas par exemple des comprimés effervescents qui permettent, dans une certaine mesure, de présenter à la fois les avantages de stabilité des formes pharmaceutiques solides et la facilité de prise d'une forme pharmaceutique liquide.

Toutefois ces formes pharmaceutiques ne sont pas universelles et présentent également des contraintes spécifiques. Dans le cas des formes effervescentes, la formulation comprend une forte quantité de sodium (carbonate ou bicarbonate de sodium) qui rend leur usage peu pertinent chez les personnes âgées souffrant d'hypertension artérielle. D'autre part la dissolution du comprimé nécessite habituellement plusieurs minutes, délai souvent considéré comme trop long par le patient.

Il subsiste donc un besoin pour un système permettant une dispensation aisée sous forme liquide des médicaments, particulièrement adaptée aux personnes âgées et aux enfants, ne présentant pas les inconvénients des formes liquides et solides connues et habituellement utilisées.

Pour y répondre la présente invention se propose d'utiliser un système de dispensation de médicaments particulier qui comprend un substrat en partie soluble, contenant au moins un principe actif, capable de changer de couleur lorsque la totalité du principe actif a été effectivement dissoute et absorbée par le patient.

On connaît des dispositifs de dispensation de molécules, notamment sous forme de pailles, qui comprennent des granulés secs solubles. On peut citer en particulier les demandes de brevet US 2008/0181932, WO-03/101.226 et US-5.718.681 qui décrivent des systèmes de délivrance se présentant sous forme de pailles permettant une absorption des granulés par aspiration d'un liquide.

Toutefois ces dispositifs, bien qu'acceptables pour un usage agro-alimentaire, ne sont pas adaptés pour l'administration de médicaments.

Tout d'abord, ils ne permettent pas de caractériser simplement la dispensation du médicament au patient.

En outre, lorsque l'utilisateur boit à travers la paille, il est physiologiquement obligé d'arrêter d'aspirer le liquide pour reprendre sa respiration. Le liquide contenu dans la paille redescend spontanément par gravité dans le verre contenant le liquide de dissolution, ce qui entraîne une perte possible du produit à ingérer contenu dans la paille. Il est alors impossible de déterminer précisément la fraction de produit ingérée par l'utilisateur.

La présente invention a donc pour objectif de pallier aux inconvénients et insuffisances de l'art antérieur.

Pour cela, l'invention vise un dispositif (10) d'administration de médicaments par voie orale, se présentant sous forme d'un corps tubulaire allongé comprenant des moyens (12, 14) aptes à faire entrer ou sortir un liquide à chaque extrémité, caractérisé en ce qu'il comprend un clapet anti-retour (16) à l'une des deux extrémités et au moins un substrat (18) constitué par au moins un support neutre (20) et une couche d'enrobage externe (22) soluble colorée contenant au moins un principe actif et présentant une coloration différente de celle du support neutre (20), le support neutre (20) étant insoluble ou le substrat (18) comprenant une couche insoluble (23) entre le support neutre (20) et la couche externe (22) cette couche (23) insoluble étant colorée avec une couleur différente de celle de la couche externe (22). Par principe actif on entend toute substance présentant des propriétés pharmacodynamiques ou thérapeutiques, y compris des bactéries probiotiques.

Par substrat on entend tout élement solide comprenant des excipients et un ou plusieurs principes actifs destiné(s) à être dissous dans un liquide. Il est composé par au moins un support neutre sur lequel est disposé au moins une couche d'enrobage soluble. Il peut se présenter par exemple sous forme de comprimé, de particule, d'agglomérat obtenu par compactage, de bille, etc.

Par couche d'enrobage on entend toute composition pharmaceutique hydrosoluble située au-dessus ou autour d'un support neutre, comprenant au moins un principe actif. La couche d'enrobage est destinée à être ingurgitée par le patient une fois dissoute dans un liquide. Il s'agit par exemple d'un pelliculage, d'un enrobage dragéifié ou d'une couche d'agglomérat.

Par support neutre on entend tout support insoluble ou faiblement soluble dans l'eau, capable de recevoir une ou plusieurs couche(s) d'enrobage(s), dépourvu de principe actif. Il est constitué d'un matériau de préférence inerte, incapable de réagir avec le milieu ou les principes actifs ou constituants pharmaceutiques qu'il reçoit. Il peut être par exemple de structure plastique polymérique et possède la compétence de permettre à la fois la fixation homogène et régulière de la couche d'enrobage tout comme sa dissolution régulière et complète dès la mise en contact avec un liquide. Il se présente par exemple sous forme de couche, de particule ou de bloc.

L'invention est maintenant décrite en détail en regard des dessins annexés sur lesquels :
- la figure 1, représente un premier mode de réalisation du dispositif selon l'invention, avec des substrats se présentant sous forme de particules,
- la figure 2, représente une vue en coupe du dispositif représenté sur la figure 1 suivant la ligne 2-2,
- les figures 3 et 4, représentent deux variantes d'un substrat sous forme de particules,
- la figure 5, représente un deuxième mode de réalisation du dispositif selon l'invention, avec un substrat se présentant sous forme d'un agglomérat,
- la figure 6, représente une vue en coupe du dispositif représenté sur la figure 5, suivant la ligne 6-6,
- la figure 7, représente un troisième mode de réalisation du dispositif selon l'invention, avec un substrat disposé sur les parois du dispositif,
- la figure 8, représente une vue en coupe du dispositif représenté sur la figure 7, suivant la ligne 8-8,
- la figure 9, représente un agrandissement de la paroi du dispositif représenté sur la figure 7,
- la figure 10A, représente un quatrième mode de réalisation du dispositif selon l'invention,
- les figures 10B à 10F, représentent un synoptique de fonctionnement de la variante de la figure 10A du dispositif selon l'invention,
- la figure 11, représentant ne vue en coupe du dispositif représenté sur la figure 10A, suivant la ligne 11-11.

L'invention vise donc un dispositif (10) d'administration de médicaments par voie orale, se présentant sous forme d'un corps tubulaire allongé comprenant des moyens (12, 14) aptes à faire entrer ou sortir un liquide à chaque extrémité, caractérisé en ce qu'il comprend un clapet anti-retour (16) à l'une des deux extrémités et au moins un substrat (18) constitué par au moins un support neutre (20) et une couche d'enrobage externe (22) soluble colorée contenant au moins un principe actif et présentant une coloration différente de celle du support neutre (20), le support neutre (20) étant insoluble ou le substrat (18) comprenant une couche insoluble (23) entre le support neutre (20) et la couche externe (22) cette couche (23) insoluble étant colorée avec une couleur différente de celle de la couche externe (22). Le clapet anti-retour 16 peut prendre toute forme adaptée permettant de maintenir un liquide dans le dispositif. Le clapet anti-retour 16 peut par exemple prendre la forme d'une bille ou d'un disque.

De façon préférée, le clapet anti-retour 16 est une bille, préférentiellement une bille en matériau dense, dépourvue de toute flottabilité dans un liquide aqueux.

Le dispositif 10 peut également comporter des moyens de blocage 34 du clapet anti-retour 16. Il peut s'agir par exemple d'un élément de rappel relié au clapet, d'une butée disposée à l'intérieur du corps tubulaire ou encore d'un rétrécissement interne de la section tubulaire du dispositif 10.

Il peut aussi comprendre au moins un insert 30 destiné à maintenir les matières insolubles à l'intérieur de la paille. Cet insert 30 peut se présenter par exemple sous forme d'une mousse à porosité ouverte ou d'une grille.

Selon un autre aspect le dispositif 10 peut présenter un bec de succion 35 au niveau des moyens de sortie 12, permettant de faciliter l'application des lèvres lors de l'utilisation du dispositif, tout en signifiant précisément et spontanément ses modalités d'emploi à l'utilisateur.

Le dispositif peut aussi se présenter sous forme d'un corps tubulaire allongé de section décroissante vers une des deux extrémités.

Le substrat 18 contenu dans le dispositif selon l'invention est constitué par au moins :
- un support neutre 20, et
- une couche 22 soluble présentant une coloration différente de celle du support neutre et contenant au moins un principe actif.

Plusieurs configurations sont possibles :
- soit le support 20 est incolore et la couche 22 est colorée,
- soit le support 20 est coloré et la couche externe 22 est colorée avec une couleur différente.

Préférentiellement la couche externe 22 soluble présente une coloration neutre, par exemple jaune orangé, et le support 20 présente une coloration verte.

Le support 20 est neutre. Il s'agit soit d'un support insoluble, soit d'un support faiblement soluble dans l'eau sur lequel est déposé une couche insoluble 23 éventuellement colorée, composé par exemple de gomme laque ou d'un autre polymère insoluble tel que l'éthylcellulose.

Dans le cas où le support interne 20 est insoluble, il peut comprendre un polymère plastique polyéthylène ou polypropylène et un agent opacifiant.

Selon une variante, il peut comprendre des billes de saccharide, de polysaccharide, de gélifiant type agar-agar, d'alginate ou d'amidon, et un agent opacifiant.

Il peut également être réalisé à partir d'un mélange de gélatine et de glycérine, classiquement utilisé pour les gélules présentées sous forme de caspules molles. Dans le cas où le support 20 est coloré, il comprend également un agent colorant. La couche externe 22 est soluble et comprend au moins un principe actif.

Selon un mode préféré de réalisation, la couche externe 22 comprend :
- un agent diluant, en particulier choisi parmi les polyols hydrate de carbone solubles type sorbitol, mannitol, saccharose, lactose ou fructose,
- un agent liant polymérique soluble tel qu'un agent cellulosique type hydrométhylcellulose, hydroxypropyleméthylcellulose, méthylcellulose, de l'alcool polyvinyique,
- de la povidone et
- au moins un principe actif.

Selon une variante, la couche externe soluble 22 comprend également un agent solubilisant, par exemple un tensio-actif comme le lauryl sulfate de sodium ou un agent type ester d'acide gras comme par exemple l'« hydrogened castor oil » et ses dérivés ou encore un composant de la famille des polysorbates.

La couche externe 22 peut également comprendre un agent édulcorant et un agent aromatisant pour améliorer le goût de la préparation après dissolution dans le liquide absorbé par le patient. La couche externe 22 colorée comprend un agent colorant. Préférentiellement cet agent colorant se présente sous la forme d'un colorant soluble ou d'un pigment fixé sur une laque d'aluminium.

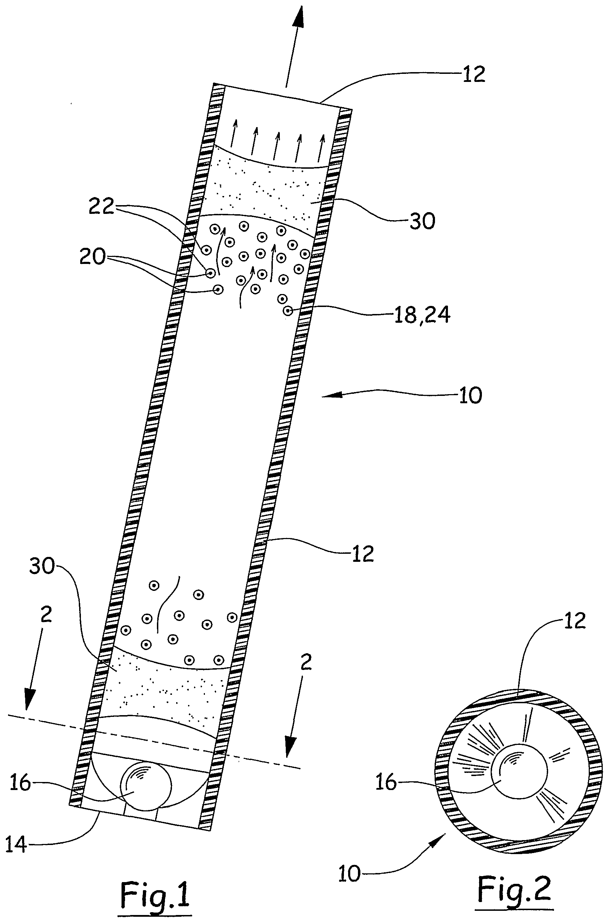

Selon un premier mode de réalisation de l'invention représenté sur la figure 1, le dispositif 10 comprend plusieurs substrats 18.

Chaque substrat se présente sous forme d'une particule 24 comprenant un support interne neutre 20, et au moins une couche d'enrobage 22 externe soluble contenant au moins un principe actif et présentant une coloration différente de celle du support neutre 20.

Soit le support interne 20 est incolore et la couche d'enrobage externe 22 est colorée, soit le support interne 20 est coloré et la couche externe d'enrobage 22 est colorée avec une couleur différente.

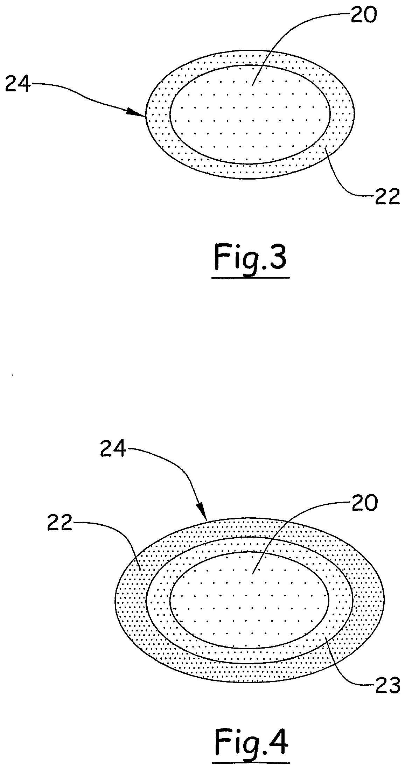

Selon une variante particulièrement adaptée, représentée sur la figure 4, la particule comprend également une couche insoluble 23 entre le support interne 20 et la couche externe 22.

Préférentiellement les particules ont une dimension comprise entre 0,8 et 4mm. Les particules colorées 24 sont fabriquées puis disposées à l'intérieur du corps tubulaire, librement, sous forme d'un bloc ou sur les parois internes du dispositif, et lorsqu'un liquide passe à l'intérieur du dispositif 10 la couche médicamenteuse 22 se trouve dissoute.

Les particules peuvent également être enfermées dans une matrice poreuse monolithique, elle-même disposée à l'intérieur du corps tubulaire de façon à ce que lorsqu'un liquide passe à l'intérieur du dispositif 10, la matrice retienne les éléments insolubles.

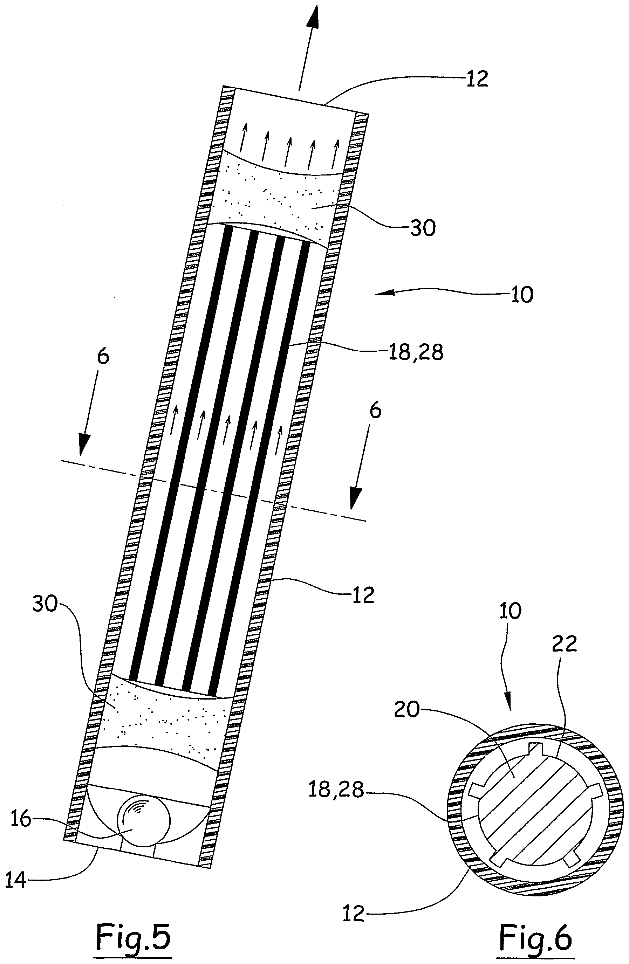

Sur la figure 5 est représenté un deuxième mode de réalisation de l'invention dans lequel le substrat 18 se présente sous forme d'un bloc 28 compact composé d'une partie interne 20 insoluble et d'une partie externe 22 soluble présentant une coloration différente de celle de la partie interne 20 et contenant au moins un principe actif.

Par bloc compact on entend un agglomérat de poudre ou un bloc homogène composé d'un polymère continu.

Le bloc compact 28 est préférentiellement un comprimé à désagrégation rapide ou un lyophilisat.

Le bloc compact 28 est fabriqué puis disposé à l'intérieur du corps tubulaire et lorsqu'un liquide passe à l'intérieur du dispositif 10 la couche externe 22 contenant le principe actif se dissout. La surface peut être augmentée par la présence de reliefs en périphérie.

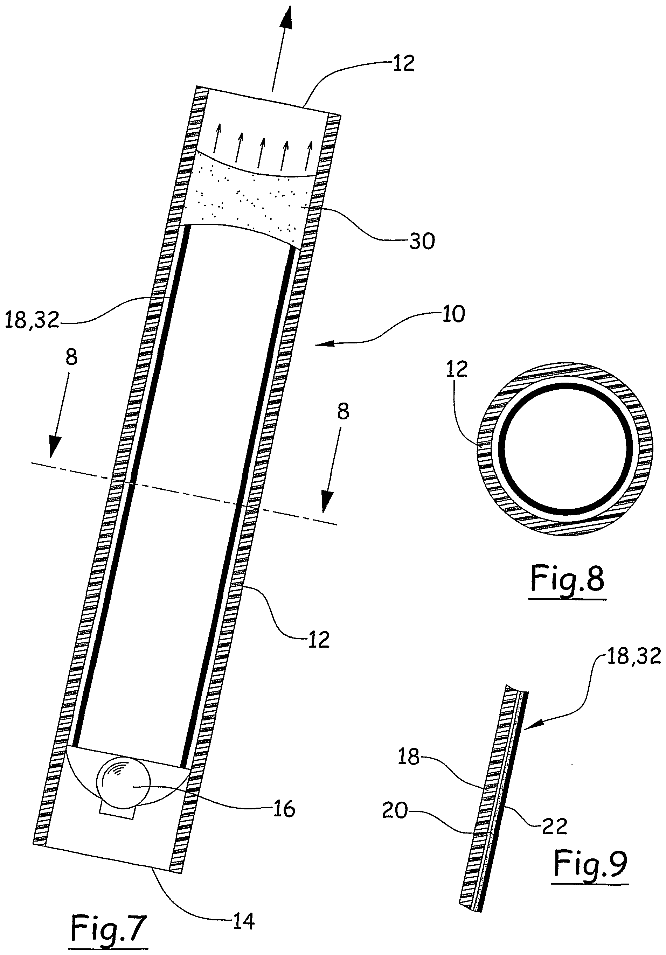

Un troisième mode de réalisation est schématisé sur la figure 7. Le substrat 18 est un revêtement interne 32 à la section tubulaire comprenant plusieurs couches, disposé sur les parois 12. Le support neutre 20 est directement en contact avec les parois du corps tubulaire ou correspond aux parois du corps tubulaire. Il est recouvert par une couche 22 soluble contenant au moins un principe actif, susceptible d'être en contact avec un liquide circulant à l'intérieur du dispositif 10, présentant une couleur différente de celle du support neutre 20.

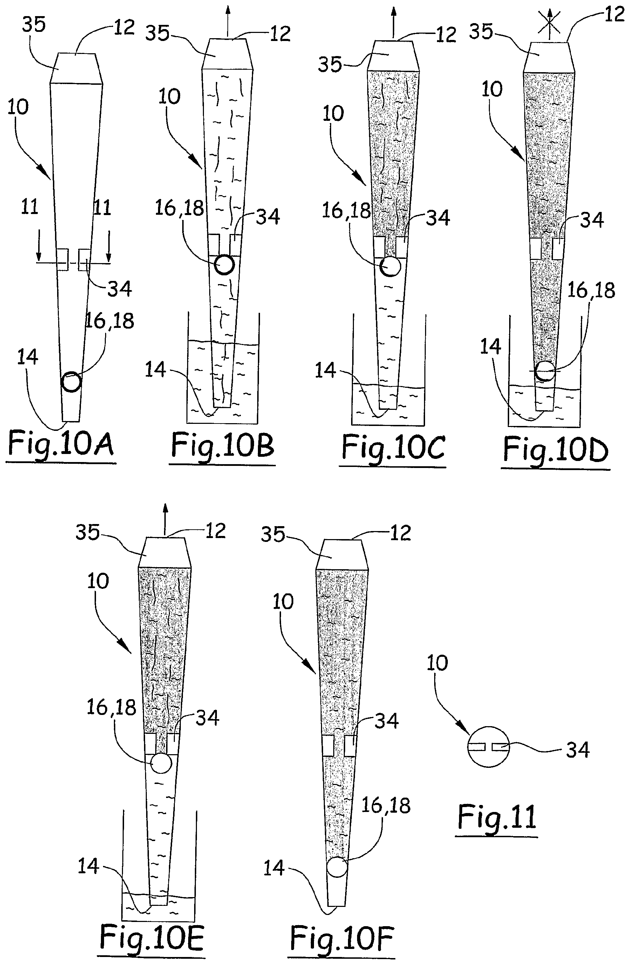

Selon un quatrième mode de réalisation simple à réaliser, représenté sur la figure 10A, le substrat 18 forme également le clapet anti-retour 16 ou est disposé sur le clapet anti-retour 16. Le dispositif 10 a une section tubulaire inférieure progressivement rétrécie vers l'extrémité 14, et comporte également des moyens de blocage supérieur 34 de la bille 16.

De façon préférée, le clapet anti-retour 16 se présente sous forme d'une bille de haute densité, comprenant un support interne 20 neutre insoluble et une couche d'enrobage externe 22 soluble présentant une coloration différente de celle du support neutre 20 et contenant au moins un principe actif.

Dans ce cas particulier, du fait de la section tubulaire inférieure progressivement rétrécie vers l'extrémité, une fois la couche externe 22 dissoute, la bille de diamètre réduit remplit toujours son rôle de clapet.

Avantageusement, le dispositif selon l'invention permet d'administrer une dose exacte et reproductible de médicament, sous forme liquide facilement absorbable.

Lorsqu'un patient aspire un liquide, comme de l'eau ou du lait par exemple, à travers le dispositif 10 selon l'invention, la couche externe médicamenteuse 22 se dissout et est ingurgitée par le patient avec le liquide. Le support neutre 20 insoluble reste dans le contenant.

Avantageusement, comme le support 20 et la couche externe 22 ont une couleur différente, lorsque la totalité du principe actif est dissoute, et donc absorbée par le patient, le contenant selon l'invention change de couleur. On peut ainsi visualiser la prise du médicament dispensé. Cet indicateur permet de s'assurer que la dose nécessaire a bien été administrée tout en évitant au patient de boire une trop grande quantité de liquide.

En outre, la présence du clapet anti-retour 16 permet d'éviter qu'une fraction indéterminée de principe actif dissous fuie dans le verre contenant le liquide de dissolution. On peut ainsi contrôler exactement la quantité de principe actif ingérée par le patient.

Sur les figures 10B à 10F les différentes étapes d'utilisation d'un mode de réalisation particulier de l'invention sont schématisées. Dans ce mode de réalisation le substrat 18 est disposé sur le clapet anti-retour 16 qui a la forme d'une bille.

Tout d'abord l'utilisateur dispose le dispositif 10 dans un verre rempli de liquide et aspire par le bec de succion 35. La dépression produite dans le tube circulaire fait monter la bille 16, ce qui libère le moyen d'entrée 14 et laisse remonter le liquide à travers le dispositif. La couche externe 22 disposée sur la bille 16 se dissout alors et le principe actif dissous dans le liquide est ingurgité par le patient. Si le patient cesse d'aspirer, la bille 16 retombe, empêchant ainsi tout retour de principe actif dissous non ingurgité dans le verre. Le patient peut ensuite aspirer à nouveau jusqu'à ce qu'il y ait un changement de couleur indiquant que la totalité du principe actif a été ingurgitée.

Le dispositif selon l'invention permet donc d'administrer facilement un médicament à un patient, notamment à une personne âgée ou à un enfant, tout en contrôlant la dispensation du médicament.

Par exemple, si la couche externe 22 soluble présente une coloration neutre jaune-orangé et le support interne 20 présente une coloration verte, lors de l'utilisation du produit, la couche externe colorée en jaune-orangé contenant le principe actif se dissout progressivement et laisse apparaître la coloration verte sous-jacente. Lorsque les support 18 et donc le dispositif 10, deviennent complètement verts, cela signifie que la totalité du principe actif médicamenteux s'est dissout et qu'il a été absorbé par le patient.

L'invention est à présent illustrée par des exemples de fabrication du premier mode de réalisation de l'invention.

### Exemple 1 : Particules avec changement de couleur jaune/vert

Dans cet exemple, le dispositif 10 selon l'invention comprend des substrats 18 sous forme de particules, chaque particule étant constituée d'un support interne neutre 20 insoluble vert, d'une couche 23 intermédiaire insoluble incolore et d'une couche d'enrobage 22 externe jaune.

La composition des différentes couches est présentée dans le tableau ci-dessous :

| | **Composant** | **Quantités** |
|---|---|---|
| **Support neutre interne (vert insoluble dans l'eau)** | Granule neutre 2 mm | 1 g |
| | Solution pelliculage verte | 0,034 g |
| | OPADRY 2 Green | |
| **Couche intermédiaire (incolore insoluble dans l'eau)** | Ethylcellulose | 0,3 g |
| **Couche d'enrobage (jaune - soluble dans l'eau)** | Paracétamol | 0,150 g |
| | Saccharose | 1,600 g |
| | Povidone | 0,045 g |
| | Talc | 0,520 g |
| | Colorant curcuma E100 | 0,060 g |
| | Arome Cranberry | 0,050 g |

Le dispositif peut être préparé selon le procédé décrit en suivant.

### Etape 1 : préparation du support neutre vert

On introduit dans une cuve en acier inoxydable de 1 litre de capacité 500 g d'eau purifiée, puis sous agitation 75 g de l'agent de pelliculage vert prêt à l'emploi type OPADRY II green ® (Hypromellose, Talc, Polydextrose, Maltodextrine, Laque FD&C Blue, Laque de jaune de quinoléine, Dioxyde de titane, triglycéride). On agite la préparation à l'aide d'un agitateur à hélice défloculante jusqu'à obtention d'une préparation se présentant sous la forme d'une suspension verte visqueuse homogène.

### Etape 2 : dépôt du support neutre vert

On introduit dans un granulateur à lit d'air fluidisé, 1000g de granules neutres de saccharose de taille comprise de 2 mm de diamètre.

On règle le débit d'air pour mettre les particules en suspension et la température d'air d'entrée à +60°C +/- 10°C

On débute la pulvérisation de la solution verte de pelliculage préparée à l'étape 1 et on poursuit le dépôt de la solution pour obtenir une masse de granule de 1,230 kg.

### Etape 3 : préparation de la solution neutre insoluble

Dans une cuve en acier inoxydable de 1000 ml de capacité, on introduit 600 ml d'eau purifiée, puis sous agitation 400g d'Ethylcellulose sous forme d'une dispersion aqueuse (équivalent à 100g de matière sèche, introduit sous forme de Surealease clear ®). On agite la préparation jusqu'à obtention d'une préparation homogène

### Etape 4 : dépôt du film incolore insoluble

Dans le granulateur à lit d'air fluidisé contenant les granules colorés verts (1,230 kg), on règle le débit d'air pour mettre les particules en suspension et la température d'air d'entrée à +60°C +/- 10°C

On pulvérise la solution de vernis incolore insoluble de pelliculage préparée à l'étape 3 et on poursuit le dépôt de la solution pour obtenir une masse de granules de 1,330 kg.

### Etape 5 : préparation de la solution médicamenteuse colorée

Dans une cuve en acier inoxydable de 1 litre de capacité, on introduit 258 ml d'eau purifiée.

Sous agitation, on introduit chronologiquement les différents composants :

| | |
|---|---|
| Paracétamol | 37,50 g |
| Saccharose | 400,00 g |
| Povidone | 11,25 g |
| Talc | 129,00 g |
| Colorant Curcuma E100 | 14,70 g |
| Arome Cranberry | 12,54 g |

On agite la solution jusqu'à obtention d'une suspension homogène.

On filtre la préparation sur un filtre de 0,1 mm d'ouverture de maille pour assurer une dispersion complète des matières en suspension

### Etape 6 : dépôt de la couche médicamenteuse colorée

Dans le granulateur à lit d'air fluidisé contenant les granules colorés verts avec le vernis isolant, on règle le débit d'air pour mettre les particules en suspension et la température d'air d'entrée à +60°C +/- 10°C

On dépose la solution préparée pour obtenir les granules médicamenteux colorés.

### Etape 7 : remplissage des pailles

On introduit un bouchon clapet anti-retour 16 dans la partie distale de la tubulure, puis on remplit les pailles avec la quantité de particules colorés pour obtenir le dosage médicamenteux adéquat.

On ferme la paille avec un bec d'aspiration 35.

### Exemple 2 : Particules avec changement de couleur rouge/vert

Dans cet exemple, le dispositif 10 selon l'invention comprend des substrats 18 sous forme de particules, chaque particule étant constituée d'un support interne neutre 20 insoluble vert, d'une couche 23 intermédiaire insoluble incolore et d'une couche d'enrobage 22 externe rouge.

La composition des différentes couches est présentée dans le tableau ci-dessous :

| | **Composant** | **Quantités** |
|---|---|---|
| **Support neutre interne (vert** - **insoluble dans l'eau)** | Granule neutre 2 mm | 1 g |
| | Solution pelliculage verte | 0 ,034 g |
| | OPADRY 2 Green | |
| **Couche intermédiaire (incolore insoluble dans l'eau)** | Ethylcellulose | 0,3 g |
| **Couche d'enrobage (rouge - soluble)** | Ibuprofène | 0,100 g |
| | Saccharose | 1 ,600 g |
| | Povidone | 0,045 g |
| | Talc | 0,520 g |
| | Aspartam | 0,050 g |
| | Colorant E120 | 0,060 g |
| | Arome Fraise | 0,050 g |

### Exemple 3 : Particules avec changement de couleur rouge/blanc

Dans cet exemple, le dispositif 10 selon l'invention comprend des substrats 18 sous forme de particules, chaque particule étant constituée d'un support interne neutre 20 insoluble incolore et d'une couche d'enrobage 22 externe rouge.

La composition des différentes couches est présentée dans le tableau ci-dessous :

| | **Composant** | **Qtes** |
|---|---|---|
| **Support neutre interne (incolore insoluble dans l'eau)** | Ethylcellulose | 0,3 g |
| | Dioxyde de titane | 0,05 g |
| **Couche d'enrobage (rouge soluble dans l'eau)** | Ibuprofène | 0,100 g |
| | Saccharose | 1,600 g |
| | Povidone | 0,045 g |
| | Talc | 0,520 g |
| | Aspartam | 0,050 g |
| | Colorant E120 | 0,060 g |
| | Arome Fraise | 0,050 g |

Bien entendu, l'invention n'est évidemment pas limitée aux exemples représentés et décrits ci-dessus, mais couvre au contraire toutes les variantes.

## Revendications

1. Dispositif (10) d'administration de médicaments par voie orale, se présentant sous forme d'un corps tubulaire allongé comprenant des moyens (12, 14) aptes à faire entrer ou sortir un liquide à chaque extrémité, **caractérisé en ce qu'**il comprend un clapet anti-retour (16) à l'une des deux extrémités et au moins un substrat (18) constitué par au moins un support neutre (20) et une couche d'enrobage externe (22) soluble colorée contenant au moins un principe actif et présentant une coloration différente de celle du support neutre (20), le support neutre (20) étant insoluble ou le substrat (18) comprenant une couche insoluble (23) entre le support neutre (20) et la couche externe (22) cette couche (23) insoluble étant colorée avec une couleur différente de celle de la couche externe (22).

2. Dispositif (10) d'administration de médicaments par voie orale selon la revendication 1, **caractérisé en ce que** le support neutre (20) est coloré.

3. Dispositif (10) d'administration de médicaments par voie orale selon l'une des revendications 1 à 2, **caractérisé en ce que** le support neutre (20) comprend un polymère plastique polyéthylène ou polypropylène, un agent opacifiant et éventuellement un agent colorant.

4. Dispositif (10) d'administration de médicaments par voie orale selon l'une des revendications 1 à 2, **caractérisé en ce que** le support neutre (20) comprend des billes de saccharide, de polysaccharide ou de gélifiant type agar-agar ou d'amidon, un agent opacifiant et éventuellement un agent colorant.

5. Dispositif (10) d'administration de médicaments par voie orale selon l'une des précédentes revendications, **caractérisé en ce que** le support neutre (20) comprend un mélange de gélatine de glycérine et éventuellement un agent colorant.

6. Dispositif (10) d'administration de médicaments par voie orale selon l'une des précédentes revendications, **caractérisé en ce que** la couche externe (22) comprend au moins un principe actif, un agent diluant, un agent de charge neutre, un agent liant, de la povidone et un agent colorant.

7. Dispositif (10) d'administration de médicaments par voie orale selon l'une des revendications 1 à 5, **caractérisé en ce que** la couche externe (22) comprend au moins un principe actif, un agent solubilisant et un agent colorant.

8. Dispositif (10) d'administration de médicaments par voie orale selon l'une des revendications 1 à 7, **caractérisé en ce que** la couche externe (22) comprend un agent colorant se présentant sous la forme d'une laque d'aluminium.

9. Dispositif (10) d'administration de médicaments par voie orale selon l'une des revendications 1 à 8, **caractérise en ce qu'**il comprend plusieurs substrats (18) se présentant sous forme de particules (24), chaque particule (24) comprenant un support interne neutre (20), et au moins une couche d'enrobage (22) externe soluble colorée contenant au moins un principe actif et présentant une coloration différente de celle du support neutre (20), le support neutre (20) étant insoluble ou le substrat (18) comprenant une couche insoluble (23) entre le support neutre (20) et la couche externe (22) cette couche (23) insoluble étant colorée avec une couleur différente de celle de la couche externe (22).

10. Dispositif (10) d'administration de médicaments par voie orale selon la revendication 9, **caractérisé en ce que** la particule a une dimension comprise entre 0,8 et 4mm.

11. Dispositif (10) d'administration de médicaments par voie orale selon l'une des revendications 1 à 8, **caractérisé en ce que** le substrat (18) se présente sous forme d'un bloc (28) compact composé d'une partie interne neutre (20), et au moins une couche d'enrobage (22) externe soluble colorée contenant au moins un principe actif et présentant une coloration différente de celle de la partie neutre (20), la partie neutre (20) étant insoluble ou le substrat (18) comprenant une couche insoluble (23) entre la partie neutre (20) et la couche externe (22) cette couche (23) insoluble étant colorée avec une couleur différente de celle de la couche externe (22).

12. Dispositif (10) d'administration de médicaments par voie orale selon l'une des revendications 1 à 8, **caractérisé en ce que** le substrat (16) est un revêtement (32) constitué par au moins un support neutre (20) correspondant aux parois du dispositif (10) ou disposé sur les parois du dispositif (10), et une couche d'enrobage externe (22) soluble colorée contenant au moins un principe actif et présentant une coloration différente de celle du support neutre, le support neutre (20) étant insoluble ou le substrat (18) comprenant une couche insoluble (23) entre le support neutre (20) et la couche externe (22) cette couche (23) insoluble étant colorée avec une couleur différente de celle de la couche externe (22).

13. Dispositif (10) d'administration de médicaments par voie orale selon l'une des précédentes revendications, **caractérisé en ce que** le substrat (18) forme également le clapet anti-retour (16) ou est disposé sur le clapet anti-retour (16).

14. Dispositif (10) d'administration de médicaments par voie orale selon l'une des précédentes revendications, **caractérisé en ce que** le clapet anti-retour (16) est une bille.

15. Dispositif (10) d'administration de médicaments par voie orale selon l'une des précédentes revendications, **caractérisé en ce qu'**il comprend des moyens (34) de blocage du clapet anti-retour (16).

16. Dispositif (10) d'administration de médicaments par voie orale selon l'une des précédentes revendications, **caractérisé en ce qu'**il se présente sous forme d'un corps tubulaire allongé de section décroissante vers une des deux extrémités.

## Patentansprüche

1. Vorrichtung (10) zur oralen Verabreichung von Medikamenten, die in der Gestalt eines länglichen rohrförmigen Körpers vorliegt und Mittel (12, 14) aufweist, die dazu geeignet sind, eine Flüssigkeit an jedem Ende eintreten oder austreten zu lassen, **dadurch gekennzeichnet, dass** diese ein Rückschlagventil (16) an einem der beiden Enden und wenigstens ein Substrat (18) aufweist, das von wenigstens einem neutralen Träger (20) und einer gefärbten löslichen äußeren Hüllschicht (22) gebildet ist, die wenigstens einen Wirkstoff enthält und eine Färbung zeigt, die sich von derjenigen des neutralen Trägers (20) unterscheidet, wobei der neutrale Träger (20) unlöslich ist oder das Substrat (18) eine unlösliche Schicht (23) zwischen dem neutralen Träger (20) und der äußeren Schicht (22) aufweist, wobei die unlösliche Schicht (23) mit einer Farbe gefärbt ist, die sich von derjenigen der äußeren Schicht (22) unterscheidet.

2. Vorrichtung (10) zur oralen Verabreichung von Medikamenten nach Anspruch 1, **dadurch gekennzeichnet, dass** der neutrale Träger (20) gefärbt ist.

3. Vorrichtung (10) zur oralen Verabreichung von Medikamenten nach einem der Ansprüche 1 bis 2, **dadurch gekennzeichnet, dass** der neutrale Träger (20) ein Plastikpolymer wie Polyethylen oder Polypropylen, ein Trübungsmittel und gegebenenfalls einen Farbstoff aufweist.

4. Vorrichtung (10) zur oralen Verabreichung von Medikamenten nach einem der Ansprüche 1 bis 2, **dadurch gekennzeichnet, dass** der neutrale Träger (20) Kugeln aus Sacchariden, Polysacchariden oder aus einem Geliermittel vom Typ Agar-Agar oder Stärke, ein Trübungsmittel und gegebenenfalls einen Farbstoff aufweist.

5. Vorrichtung (10) zur oralen Verabreichung von Medikamenten nach einem der vorhergehenden Ansprüche, **dadurch gekennzeichnet, dass** der neutrale Träger (20) eine Mischung von Glyzeringelatine und gegebenenfalls einen Farbstoff aufweist.

6. Vorrichtung (10) zur oralen Verabreichung von Medikamenten nach einem der vorhergehenden Ansprüche, **dadurch gekennzeichnet, dass** die äußere Schicht (22) wenigstens einen Wirkstoff, ein Verdünnungsmittel, einen neutralen Füllstoff, ein Verbindungsmittel, Providon und einen Farbstoff aufweist.

7. Vorrichtung (10) zur oralen Verabreichung von Medikamenten nach einem der Ansprüche 1 bis 5, **dadurch gekennzeichnet, dass** die äußere Schicht (22) wenigstens einen Wirkstoff, ein Lösungsmittel und einen Farbstoff aufweist.

8. Vorrichtung (10) zur oralen Verabreichung von Medikamenten nach einem der Ansprüche 1 bis 7, **dadurch gekennzeichnet, dass** die äußere Schicht (22) einen Farbstoff aufweist, der in der Gestalt von Aluminiumpigmenten vorliegt.

9. Vorrichtung (10) zur oralen Verabreichung von Medikamenten nach einem der Ansprüche 1 bis 8, **dadurch gekennzeichnet, dass** diese mehrere Substrate (18) umfasst, die in der Gestalt von Teilchen (24) vorliegen, wobei die Teilchen (24) jeweils einen inneren neutralen Träger (20) und wenigstens eine gefärbte lösliche äußere Hüllschicht (22) aufweist, die wenigstens einen Wirkstoff enthält und eine Färbung aufweist, die sich von derjenigen des neutralen Trägers (20) unterscheidet, wobei der neutrale Träger (20) unlöslich ist oder das Substrat (18) eine unlösliche Schicht (23) zwischen dem neutralen Träger (20) und der äußeren Schicht (22) aufweist, wobei diese unlösliche Schicht (23) mit einer Farbe gefärbt ist, die sich von derjenigen der äußeren Schicht (22) unterscheidet.

10. Vorrichtung (10) zur oralen Verabreichung von Medikamenten nach Anspruch 9, **dadurch gekennzeichnet, dass** das Teilchen Abmessungen zwischen 0,8 und 4 mm hat.

11. Vorrichtung (10) zur oralen Verabreichung von Medikamenten nach einem der Ansprüche 1 bis 8, **dadurch gekennzeichnet, dass** das Substrat (18) in der Gestalt eines kompakten Blocks (28) vorliegt, der aus einem neutralen inneren Teil (20) und wenigstens einer gefärbten löslichen äußeren Hüllschicht (22) zusammengesetzt ist, die wenigstens einen Wirkstoff enthält und eine Färbung aufweist, die sich von derjenigen des neutralen Teils (20) unterscheidet, wobei der neutrale Teil (20) unlöslich ist oder das Substrat (18) eine unlösliche Schicht (23) zwischen dem neutralen Teil (20) und der äußeren Schicht (22) aufweist, wobei die unlösliche Schicht (23) mit einer Farbe gefärbt ist, die sich von derjenigen der äußeren Schicht (22) unterscheidet.

12. Vorrichtung (10) zur oralen Verabreichung von Medikamenten nach einem der Ansprüche 1 bis 8, **dadurch gekennzeichnet, dass** das Substrat (18) eine Verkleidung (32) ist, die von wenigstens einem neutralen Träger (20) gebildet ist, der den Wänden der Vorrichtung (10) entspricht oder auf den Wänden der Vorrichtung (10) angeordnet ist, und von einer gefärbten löslichen äußeren Hüllschicht (22) gebildet ist, die wenigstens einen Wirkstoff enthält und eine Färbung aufweist, die sich von derjenigen des neutralen Trägers unterscheidet, wobei der neutrale Träger (20) unlöslich ist oder das Substrat (18) eine unlösliche Schicht (23) zwischen dem neutralen Träger (20) und der äußeren Schicht (22) aufweist, wobei diese unlösliche Schicht (23) mit einer Farbe gefärbt ist, die sich von derjenigen der äußeren Schicht (22) unterscheidet.

13. Vorrichtung (10) zur oralen Verabreichung von Medikamenten nach einem der vorhergehenden Ansprüche, **dadurch gekennzeichnet, dass** das Substrat (18) auch das Rückschlagventil (16) bildet oder auf dem Rückschlagventil (16) angeordnet ist.

14. Vorrichtung (10) zur oralen Verabreichung von Medikamenten nach einem der vorhergehenden Ansprüche, **dadurch gekennzeichnet, dass** das Rückschlagventil (16) eine Kugel ist.

15. Vorrichtung (10) zur oralen Verabreichung von Medikamenten nach einem der vorhergehenden Ansprüche, **dadurch gekennzeichnet, dass** diese Mittel (34) zum Blockieren des Rückschlagventils (16) aufweist.

16. Vorrichtung (10) zur oralen Verabreichung von Medikamenten nach einem der vorhergehenden Ansprüche, **dadurch gekennzeichnet, dass** diese in der Gestalt eines länglichen rohrförmigen Körpers mit zu einem der beiden Enden abnehmendem Querschnitt vorliegt.

## Claims

1. A device (10) for the oral administration of drugs, in the form of a elongate tubular body comprising means (12, 14) able to make a liquid enter or leave at each end, **characterised in that** it comprises a non-return valve (16) at one of the two ends and at least one substrate (18) formed by at least one neutral support (20) and a coloured soluble external coating layer (22) containing at least one active principle and having a colouring different from that of the neutral support (20), the neutral support (20) being insoluble or the substrate (18) comprising an insoluble layer (23) between the neutral support (20) and the external layer (22), this insoluble layer (23) being coloured with a colour different from that of external layer (22).

2. A device (10) for the oral administration of drugs according to claim 1, **characterised in that** the neutral support (20) is coloured.

3. A device (10) for the oral administration of drugs according to claims 1 to 2, **characterised in that** the neutral support (20) comprises a polyethylene or polypropylene plastics polymer, an opacifier and optionally a dye.

4. A device (10) for the oral administration of drugs according to claims 1 to 2, **characterised in that** the neutral support (20) comprises beads of saccharide, polysaccharide or gelling agent of the agar-agar or starch type, an opacifier and optionally a dye.

5. A device (10) for the oral administration of drugs according to any of the preceding claims, **characterised in that** the neutral support (20) comprises a mixture of gelatin, glycerine and optionally a dye.

6. A device (10) for the oral administration of drugs according to any of the preceding claims, **characterised in that** the external layer (22) comprises at least one active principle, a diluent, a neutral filler, a binder, povidone and a dye.

7. A device (10) for the oral administration of drugs according to any of claims 1 to 5, **characterised in that** the external layer (22) comprises at least one active principle, a solubilising agent and a dye.

8. A device (10) for the oral administration of drugs according to any of claims 1 to 7, **characterised in that** the external layer (22) comprises a dye in the form of an aluminium lacquer.

9. A device (10) for the oral administration of drugs according to any of claims 1 to 8, **characterised in that** it comprises a plurality of substrates (18) in the form of particles (24), each particle (24) comprising a neutral internal support (20), and at least one coloured soluble external coating layer (22) containing at least one active principle and having a colouring different from that of the neutral support (20), the neutral support (20) being insoluble or the substrate (18) comprising an insoluble layer (23) between the neutral support (20) and the external layer (22), this insoluble layer (23) being coloured with a colour different from that of the external layer (22).

10. A device (10) for the oral administration of drugs according to claim 9, **characterised in that** the particle has a size of between 0.8 and 4 mm.

11. A device (10) for the oral administration of drugs according to any of claims 1 to 8, **characterised in that** the substrate (18) is in the form of a compact block (28) composed of a neutral internal part (20), and at least one coloured soluble external coating layer (22) containing at least one active principle and having a colouring different from that of the neutral part (20), the neutral part (20) being insoluble or the substrate (18) comprising an insoluble layer (23) between the neutral part (20) and the external layer (22), this insoluble layer (23) being coloured with a colour different from that of the external layer (22).

12. A device (10) for the oral administration of drugs according to any of claims 1 to 8, **characterised in that** the substrate (18) is a cladding (32) comprising at least one neutral support (20) corresponding to the walls of the device (10) or disposed on the walls of the device (10), and a coloured soluble external coating layer (22) containing at least one active principle and having a colouring different from that of the neutral support (20), the neutral support (20) being insoluble or the substrate (18) comprising an insoluble layer (23) between the neutral support (20) and the external layer (22), this insoluble layer (23) being coloured with a colour different from that of the external layer (22).

13. A device (10) for the oral administration of drugs according to any of the preceding claims, **characterised in that** the substrate (18) also forms the non-return valve (16) or is disposed on the non-return valve (16).

14. A device (10) for the oral administration of drugs according to any of the preceding claims, **characterised in that** the non-return valve (16) is a ball.

15. A device (10) for the oral administration of drugs according to any of the preceding claims, **characterised in that** it comprises means (34) for blocking the non-return valve (16).

16. A device (10) for the oral administration of drugs according to any of the preceding claims, **characterised in that** it is in the form of an elongate tubular body with a cross section decreasing towards one of the two ends.
